# EUROPEAN PATENT SPECIFICATION

(11) **EP 1 094 847 B1**
(45) Date of publication and mention of the grant of the patent: **31.03.2004**
(21) Application number: 99934594.5
(22) Date of filing: 05.07.1999
(51) Int. Cl.: A61L 2/20, A61L 11/00, A61L 101/10

(54) **A METHOD FOR DISINFECTING AND STERILIZING MICROBIAL CONTAMINATED MATERIALS**
VERFAHREN ZUM DESINFINZIEREN UND STERILISIEREN VON MIKROBIELL KONTAMINIERTEN MATERIALEN
PROCEDE DE DESINFECTION ET DE STERILISATION DU MATERIEL CONTAMINE PAR DES MICROBES

(30) Priority: 10.07.1998 US 113699
(43) Date of publication of application: 02.05.2001
(73) Proprietor: Box 03 International, 3073 Gümlingen (CH)
(72) Inventor: DUROSELLE, Patrick, F-69008 Lyon (FR)
(74) Representative: BOVARD AG - Patentanwälte
(86) International application number: PCT/EP1999/004723
(87) International publication number: WO 2000/002595

(56) References cited:
- WO-A-94/21120
- US-A- 5 567 444
- US-A- 5 674 450
- US-A- 5 700 426

## Description

The present invention relates to a method for disinfecting and sterilizing microbial contaminated materials, e.g. medical devices, instruments, waste from laboratories or hospitals comprising blood or blood components or other biological material under use of a combination of *in situ* generated peracetic acid and ozone.

For disinfecting of medical devices, working tools or infectious waste material in the medical field, several methods using ozone are suggested according to prior art. The main problem of known methods has been the long period for treating the material containing pathogenic agents such as bacteria and viruses. With a shorter treatment problems occurred with material containing biological liquids such as blood or components thereof (probably due to hemoglobin presence) because an amount of resistant spores survived. It was found that an improvement can be attained if a combination of ozone and peracetic acid was used in a humidified gaseous phase.

WO-A-94 21120 discloses a process for sterilising a load of items by introducing it into a sterilisation chamber, introducing peracid sterilant vapour and reducing the pressure below atmospheric pressure, introducing a non-interfering gas and increasing the pressure in the chamber; ozone is explicitly not used. According to the specification peracid vapor (i.a. peracetic acid vapour) is used instead of ozone or hydrogen peroxide because ozone attacks a number of structural materials such as natural gum rubber, several metals and some common plastics, and because hydrogen peroxide attacks cellulose wrapping materials.

US-A-5 567 444 describes a cleaning and sanitising method for solid surfaces. In this method, in a first step, the surface is treated with an ozonized cleaning composition at a basic pH of about 8. In a second step, the surface is contacted with an aqueous composition, comprising an effective amount of hydrogen peroxide, a C₁-C₁₀ peroxyaliphatic carboxylic acid or a mixture thereof. In this document, the combination of a peracid with ozone is disclosed, however, not in an evacuated vessel.

None of the above-mentioned documents discloses a method for disinfecting waste materials in a sterilisation chamber with a combination of peracetic acid and ozone under reduced pressure.

According US-A-5,374,394 peracetic acid has been used in liquid form for decontaminating medical wastes.

Another approach is described in the document JP-A-7136236 wherein hot vapor is injected into a sterilization chamber. The use of a hot oxidizing agent can be irritating and dangerous in the case of a defect in the chamber.

The present invention is related to a method for disinfecting and sterilizing microbial contaminated or infectious materials, e.g. medical instruments, waste from medical laboratories or hospitals.

The method comprises the steps:
(a) loading the material for disinfecting and sterilizing into a vacuum-proof sterilization chamber;
(b) introducing liquid hydrogen peroxide into the sterilization chamber so as to penetrate the material;
(c) introducing liquid acetic acid into the sterilization chamber so as to penetrate the material;
(d) evacuating gas from the sterilization chamber so as to evaporate the liquid at least partially;
(e) introducing gaseous ozone into the sterilization chamber;
(f) treating the material in the sterilization chamber for a sufficient time period so as to disinfect and sterilize the materials.

Alternatively, the method comprises the steps:
(a) loading the material for disinfecting and sterilizing into a vacuum-proof sterilization chamber;
(b) introducing liquid hydrogen peroxide into the sterilization chamber in an ampoule having such properties that after an evacuation it bursts with release of its content;
(c) introducing liquid acetic acid into the sterilization chamber in an ampoule having such properties that after an evacuation it bursts with release of its content;
(d) evacuating gas from the sterilization chamber so that the two separate ampoules of step (b) and (c) burst with release of their content so as to evaporate the liquid at least partially;
(e) introducing gaseous ozone into the sterilization chamber;
(f) treating the material in a sterilization chamber for a sufficient time period so as to disinfect and sterilize the materials.

Alternatively, the method comprises the steps:
(a) loading the material for disinfecting and sterilizing into a vacuum-proof sterilization chamber.
(b) and (c) introducing liquid hydrogen peroxide and liquid acetic acid in a container having at least two compartments being constructed in such a manner that their contents are released during an evacuation
(d) evacuating gas from the sterilization chamber so that the two separate compartments of step (b) and (c) release their content so as to evaporate the liquid at least partially;
(e) introducing gaseous ozone into the sterilization chamber;
(f) treating the material in a sterilization chamber for a sufficient time period so as to disinfect and sterilize the materials.

The method can be carried out in treatment devices as described in the patent documents EP-A- 0 664 715 and EP-A-0 761 237, which can be adapted to the method according to present invention.

The method of the present invention is carried out, as a rule, at a temperature of about 15 °C to 35 °C and preferably at ambient temperature.

The method of the invention can be carried out in two or more cycles by feeding at least two times hydrogen peroxide, the acetic acid and/or ozone into the sterilization chamber.

The molar ratio of acetic acid to ozone according to the invention is as a rule about 3/1 to 1/3 and the humidity in the chamber is at least 10 %.

The infectious waste material treated according to the method of the present invention can be solid material or a mixture of solid and liquid material. For disinfecting waste material safely, it is ground or broken up in other wise before, during or after the treatment with the disinfecting agents (hydrogen peroxide and acetic acid).

In the method of the invention a stable, storable and shippable disinfecting and sterilizing combination of agents is used together with ozone. The combination possesses improved properties and comprises a two-part system: the first part consists of a mixture of acetic acid and water, and the second consists of hydrogen peroxide and water. In this solution the peracetic acid is formed *in situ*. With this measure it is possible to take advantage of the outstanding disinfecting and sterilizing properties of peracetic acid without exposure to the danger of explosion and to the strong irritating properties to the skin and the eyes. The application of the peracetic acid forming combination with ozone is carried out in a gaseous phase under reduced pressure (pressure < atmospheric pressure) and at ambient temperature. Hydrogen peroxide reacts with acetic acid to form of peracetic acid and water:

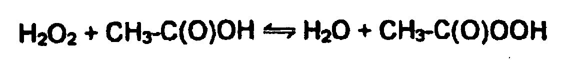

This is an equilibrium which can be shifted if the reaction takes place in the presence of a catalytic amount of acid (e.g. sulfuric acid). After termination of the disinfecting process the remaining ozone is destroyed, and, if necessary, the acid can be neutralized.

The present invention is illustrated with sterilizing tests carried out in a sterilizing apparatus of the type "BOX 03" of Carbagas Aktiengesellschaft CH 3097 Liebefeld (Switzerland). This apparatus was designed for disinfecting infectious waste material. Normally in this device the waste material is ground , evacuated and treated one or several times with gaseous ozone in a sterilizing chamber. According to the present invention *in situ*-formed peracetic acid is used additionally. For comparison, instead of peracetic acid only hydrogen peroxide was used. For the test, pads loaded with infected sheep's blood were used. The results are listed in the table below.

**Table:**

| Number of viable and sporular bacterial forms destroyed in a sterilization chamber on a pad in the presence of blood of a sheep (100 µl per germ-carrier) | | | | | |
|---|---|---|---|---|---|
| **micro-organism** | **Number of cycles** | **Formation time** | **Compound 1** | **Compound 2** | **Reduction of micro-organisms** |
| *S*. *aureus* +100µl of sheep's blood | 3 | 900 s | H₂O₂ | - | 3.87 log₁₀ |
| *S. aureus* +100µl of sheep's blood | 3 | 900 s | H₂O₂ | CH₃COOH | 7.90 log₁₀ |
| *S. aureus* +100µl of sheep's blood | 3 | 900 s | H₂O₂ | CH₃COOH | <8.11 log₁₀ |
| *E.hirae* +100µl of sheep's blood | 3 | 900 s | H₂O₂ | - | 2.23 log₁₀ |
| *E.hirae* +100µl of sheep's blood | 4 | 600 s | H₂O₂ | CH₃COOH | ≥7 log₁₀ |
| *E.hirae* +100µl of sheep's blood | 3 | 900 s | H₂O₂ | CH₃COOH | <7.8 log₁₀ |
| *E.coli* +100µl of sheep's blood | 3 | 900 s | H₂O₂ | CH₃COOH | <7.69 log₁₀ |
| *E.coli* +100µl of sheep's blood | 5 | 480 s | H₂O₂ | - | 1.38 log₁₀ |
| *E.coli* +100µl of sheep's blood | 3 | 900 s | H₂O₂ | CH₃COOH | ≥8 log₁₀ |
| *E.coli* +100µl of sheep's blood | 3 | 900 s | H₂O₂ | CH₃COOH | <8 log₁₀ |
| *P. aeruginosa* +100µl of sheep's blood | 5 | 480 s | H₂O₂ | - | 1.47 log₁₀ |
| *P. aeruginosa* +100µl of sheep's blood | 4 | 900 s | H₂O₂ | CH₃COOH | 7 log₁₀ |
| *P. aeruginosa* +100µl of sheep's blood | 3 | 900 s | H₂O₂ | CH₃COOH | ≥8.25 log₁₀ |
| *P. aeruginosa* +100µl of sheep's blood | 3 | 900 s | H₂O₂ | CH₃COOH | 8.47 log₁₀ |
| *M.smegmatis* +100µl of sheep's blood | 3 | 900 s | H₂O₂ | - | 2.47 log₁₀ |
| *M.smegmatis* +100µl of sheep's blood | 4 | 600 s | H₂O₂ | CH₃COOH | ≤7 log₁₀ |
| *M.smegmatis* +100µl of sheep's blood | 3 | 900 s | H₂O₂ | CH₃COOH | ≤7.6 log₁₀ |
| *M.smegmatis* +100µl of sheep's blood | 3 | 900 s | H₂O₂ | CH₃COOH | ≤8 log₁₀ |
| *C. albicans* +100µl of sheep's blood | 3 | 900 s | H₂O₂ | - | 1.8 log₁₀ |
| *C. albicans* +100µl of sheep's blood | 3 | 900 s | H₂O₂ | CH₃COOH | ≥8.04 log₁₀ |
| *C. albicans* +100µl of sheep's blood | 3 | 900 s | H₂O₂ | CH₃COOH | ≥8.04 log₁₀ |
| *C. albicans* +100µl of sheep's blood | 3 | 900 s | H₂O₂ | CH₃COOH | <8 log₁₀ |
| *B.subtilis* spores +100µl of sheep's blood | 3 | 480 s | H₂O₂ | - | 3 log₁₀ |
| *B.subtilis* spores +100µl of sheep's blood | 5 | 600 s | H₂O₂ | CH₃COOH | ≥3 log₁₀ |
| *B.subtilis* spores +100µl of sheep's blood | 4 | 600 s | H₂O₂ | CH₃COOH | ≥6 log₁₀ |
| *B.subtilis* spores +100µl of sheep's blood | 4 | 900 s | H₂O₂ | CH₃COOH | ≥6 log₁₀ |
| *B.subtilis* spores +100µl of sheep's blood | 3 | 900 s | H₂O₂ | CH₃COOH | 4 log₁₀ |
| *B.stearoth* spores +100µl of sheep's blood | 5 | 480 s | H₂O₂ | - | <4 log₁₀ |
| *B.stearoth* spores +100µl of sheep's blood | 4 | 600 s | H₂O₂ | CH₃COOH | ≥5 log₁₀ |
| *B.stearoth* spores +100µl of sheep's blood | 4 | 600 s | H₂O₂ | CH₃COOH | ≥5 log₁₀ |
| *B.stearoth* spores +100µl of sheep's blood | 3 | 900 s | H₂O₂ | CH₃COOH | ≥5 log₁₀ |

The table shows that the germicide potency of the method according to the invention is markedly improved in comparison with the method where only the germicide compound H₂O₂ is used in combination with O₃.

## Claims

1. A method for disinfecting and sterilizing microbial contaminated or infectious materials, e.g. medical instruments, waste from medical laboratories or hospitals, the steps comprising:
(a) loading the material for disinfecting and sterilizing into a vacuum-proof sterilization chamber
(b) introducing liquid hydrogen peroxide into the sterilization chamber so as to penetrate the material;
(c) introducing liquid acetic acid into the sterilization chamber so as to penetrate the material;
(d) evacuating gas from the sterilization chamber so as to evaporate the liquid at least partially;
(e) introducing gaseous ozone into the sterilization chamber;
(f) treating the material in a sterilization chamber for a sufficient time period so as to disinfect and sterilize the materials.

2. A method for disinfecting and sterilizing microbial contaminated or infectious materials, e.g. medical instruments, waste from medical laboratories or hospitals, the steps comprising:
(a) loading the material for disinfecting and sterilizing into a vacuum-proof sterilization chamber;
(b) introducing liquid hydrogen peroxide into the sterilization chamber in an ampoule having such properties that after an evacuation it bursts with release of its content;
(c) introducing liquid acetic acid into the sterilization chamber in an ampoule having such properties that after an evacuation it bursts with release of its content;
(d) evacuating gas from the sterilization chamber so the two separate ampoules of step (b) and (c) burst with release of their content to evaporate the liquid at least partially;
(e) introducing gaseous ozone into the sterilization chamber;
(f) treating the material in a sterilization chamber for a sufficient time period so as to disinfect and sterilize the materials.

3. A method for disinfecting and sterilizing microbial contaminated or infectious materials, e.g. medical instruments, waste from medical laboratories or hospitals, the steps comprising:
(a) loading the material for disinfecting and sterilizing into a vacuum-proof sterilization chamber;
(b) and (c) introducing liquid hydrogen peroxide and liquid acetic acid in a container having at least two compartments being constructed in such a manner that their contents are reteased during an evacuation;
(d) evacuating gas from the sterilization chamber so that the two separate compartments of step (b) and (c) release their content so as to evaporate the liquid at least partially;
(e) introducing gaseous ozone into the sterilization chamber;
(f) treating the material in a sterilization chamber for a sufficient time period so as to disinfect and sterilize the materials.

4. The method of claim 1 wherein the steps (b) and (c) are carried out in any sequence under *in situ* generation of peracetic acid.

5. The method of claim 1 wherein the steps (b) and (c) are carried out simultaneously under *in situ* generation of peracetic acid.

6. The method according to one of the claims 1 to 5 wherein the method is carried out at a temperature of about 15 °C to 35 °C.

7. The method of claim 6 wherein the method is carried out at ambient temperature.

8. The method of claim 3 wherein the container has a compartment with a catalyst for shifting the equilibrium of the reaction equation 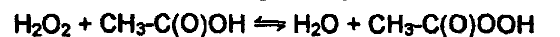 to the right, which catalyst is released at the same time as the hydrogen peroxide and the acetic acid.

9. The method of claim 8 wherein the catalyst is sulfuric acid.

10. The method according to one of the claims 1 to 9 wherein in step (f) the ozone and the formed peracetic acid are present in the vapor phase.

11. The method according to one of the claims 1 to 10 wherein the steps (b) to (e) are carried out at least two times.

12. The method according to one of the claim 1 to 11 wherein at the sterilization chamber feeding means for feeding hydrogen peroxide, acetic acid and ozone are disposed.

13. The method according to one of the claims 1 to 12 wherein the molar ratio of acetic acid to ozone is 3/1 to 1/3.

14. The method according to one of the claims 1 to 13 wherein the humidity in step (e) or (f) is at least 10 %.

15. The method according to one of the claims 1 to 14 wherein the material is infectious waste in a solid or liquid state.

16. The method according to one of the claims 2 to 15 for treating contaminated or infectious waste wherein the waste is ground before, during or after step (b) or (c).

17. The method according to one of the claims 1 to 16 wherein step (e) is carried out at least twice.

## Patentansprüche

1. Verfahren zur Desinfektion von mikrobiell kontaminiertem oder infektiösem Material, z.B. medizinische Instrumente, Abfall von medizinischen Laboratorien oder Spitälern, enthaltend die Schritte:
(a) beladen einer vakuumfesten Sterilisationskammer mit dem zu desinfizierenden und zu sterilisierenden Material;
(b) einleiten von flüssigem Wassserstoffperoxid in die Sterilisationskammer, derart, dass es in das Material eindringen kann;
(c) einleiten von flüssiger Essigsäure in die Sterilisationskammer derart, dass sie in das Material eindringen kann;
(d) evakuieren des Gases aus der Sterilisationskammer, derart, dass die Flüssigkeit zumindest teilweise verdampft;
(e) einleiten von gasförmigem Ozon in die Sterilisationskammer;
(f) behandeln des Materials in der Sterilisationskammer während einer genügenden Zeitperiode um die Materialien zu desinfizieren und zu sterilisieren.

2. Verfahren zur Desinfektion von mikrobiell kontaminiertem oder infektiösem Material, z.B. medizinische Instrumente, Abfall von medizinischen Laboratorien oder Spitälern, enthaltend die Schritte:
(a) beladen einer vakuumfesten Sterilisationskammer mit dem zu desinfizierenden und zu sterilisierenden Material;
(b) einführen von flüssigem Wasserstoffperoxid in die Sterilisationskammer in einer Ampulle, die solche Eigenschaften besitzt, dass sie im Vakuum unter Freigabe ihres Inhaltes zerbricht;
(c) einführen von flüssiger Essigsäure in die Sterilisationskammer in einer Ampulle, die solche Eigenschaften besitzt, dass sie im Vakuum unter Freigabe ihres Inhaltes zerbricht;
(d) evakuieren des Gases aus der Sterilisationskammer, derart, dass die beiden separaten Ampullen der Schritte (b) und (c) unter Freigabe ihres Inhalts zerbrechen, so dass die Flüssigkeit zumindest teilweise verdampft;
(e) einleiten von gasförmigem Ozon in die Sterilisationskammer;
(f) behandeln des Materials in der Sterilisationskammer während einer genügenden Zeitperiode um die Materialien zu desinfizieren und zu sterilisieren.

3. Verfahren zur Desinfektion von mikrobiell kontaminiertem oder infektiösem Material, z.B. medizinische Instrumente, Abfall von medizinischen Laboratorien oder Spitälern, enthaltend die Schritte:
(a) beladen einer vakuumfesten Sterilisationskammer mit dem zu desinfizierenden und zu sterilisierenden Material;
(b) und (c) einführen von flüssigem Wasserstoffperoxid und von flüssiger Essigsäure in einem Behälter, welcher zumindest zwei Abteile besitzt in die Sterilisationskammer, wobei der Behälter so konstruiert ist, dass er während der Evakuierung seinen Inhalt freigibt;
(d) evakuieren des Gases aus der Sterilisationskammer, derart, dass die zwei separaten Abteile der Schritte (b) und (c) ihren Inhalt freigeben, so dass die Flüssigkeit zumidest teilweise verdampft;
(e) einleiten von gasförmigem Ozon in die Sterilisationskammer;
(f) behandeln des Materials in der Sterilisationskammer während einer genügenden Zeitperiode um die Materialien zu desinfizieren und zu sterilisieren.

4. Verfahren gemäss Anspruch 1, worin die Schritte (b) und (c) in irgend einer Reihenfolge durchgeführt werden unter einer *in situ*-Bildung von Peressigsäure.

5. Verfahren gemäss Anspruch 1, worin die Schritte (b) und (c) gleichzeitig durchgeführt werden, unter einer *in situ*-Bildung von Peressigsäure.

6. Verfahren gemäss einem der Ansprüche 1 bis 5, worin das Verfahren bei einer Temperatur von etwa 15 °C bis 35 °C durchgeführt wird.

7. Verfahren gemäss Anspruch 6, worin das Verfahren bei Umgebungstemperatur durchgeführt wird.

8. Verfahren gemäss Anspruch 3, worin der Behälter ein Abteil mit einem Katalysator besitzt, für die Verschiebung des folgenden Gleichgewichts nach rechts,
H₂O₂ + CH₃-C(O)OH ⇆ H₂O + CH₃-C(O)OOH
wobei der Katalysator gleichzeitig wie das Wasserstoffperoxid und die Essigsäure freigesetzt wird.

9. Verfahren gemäss Anspruch 8, worin der Katalysator Schwefelsäure ist.

10. Verfahren gemäss einem der Ansprüche 1 bis 9, worin im Schritt (f) das Ozon und die gebildete Peressigsäure in der Dampfphase vorliegen.

11. Verfahren gemäss einem der Ansprüche 1 bis 10, worin die Schritte (b) und (e) mindesten zwei Mal durchgeführt werden.

12. Verfahren gemäss einem der Ansprüche 1 bis 11, worin in der Sterilisationskammer Zufuhrmittel für die Zufuhr von Wasserstoffperoxid, Essigsäure und Ozon angeordnet sind.

13. Verfahren gemäss einem der Ansprüche 1 bis 12 worin das Molverhältnis von Essigsäure zu Ozon 3/1 bir 1/3 beträgt.

14. Verfahren gemäss einem der Ansprüche 1 bis 14, worin die Feuchtigkeit in den Schritten (e) oder (f) mindestens 10 % beträgt.

15. Verfahren gemäss einem der Ansprüche 1 bis 14, worin das Material infektiöser Abfall in festem oder flüssigem Zustand ist.

16. Verfahren gemäss einem der Ansprüche 1 bis 15 für die Behandlung von kontaminiertem oder infektiösem Abfall, worin der Abfall vor, während oder nach den Schritten (b) oder (c) gemahlen wird.

17. Verfahren gemäss einem der Ansprüche 1 bis 16, worin der Schritt (e) mindestens zwei Mal durchgeführt wird.

## Revendications

1. Procédé pour désinfecter et stériliser des matériels infectieux ou contaminés par des microbes, par exemple des instruments médicaux, des déchets de laboratoires médicaux ou d'hôpitaux, les étapes comprenant :
(a) la mise en place du matériel à désinfecter et à stériliser dans une chambre de stérilisation étanche au vide ;
(b) l'introduction de peroxyde d'hydrogène liquide dans la chambre de stérilisation, de façon qu'il pénètre dans le matériel ;
(c) l'introduction d'acide acétique liquide dans la chambre de stérilisation, de façon qu'il pénètre dans le matériel ;
(d) l'élimination du gaz de la chambre de stérilisation de façon à provoquer une évaporation au moins partielle du liquide ;
(e) l'introduction d'ozone gazeux dans la chambre de stérilisation ;
(f) le traitement du matériel dans une chambre de stérilisation pendant un laps de temps suffisant pour désinfecter et stériliser les matériels.

2. Procédé pour désinfecter et stériliser des matériels infectieux ou contaminés par des microbes, par exemple des instruments médicaux, des déchets de laboratoires médicaux ou d'hôpitaux, les étapes comprenant :
(a) la mise en place du matériel à désinfecter et à stériliser dans une chambre de stérilisation étanche au vide ;
(b) l'introduction de peroxyde d'hydrogène liquide dans la chambre de stérilisation, dans une ampoule ayant des propriétés lui permettant d'éclater, après évacuation, avec libération de son contenu ;
(c) l'introduction d'acide acétique liquide dans la chambre de stérilisation, dans une ampoule ayant des propriétés lui permettant d'éclater, après évacuation, avec libération de son contenu ;
(d) l'évacuation du gaz de la chambre de stérilisation, de façon que les deux ampoules distinctes des étapes (b) et (c) éclatent avec libération de leur contenu, de façon à provoquer une évaporation au moins partielle du liquide ;
(e) l'introduction d'ozone gazeux dans la chambre de stérilisation ;
(f) le traitement du matériel dans une chambre de stérilisation pendant un laps de temps suffisant pour désinfecter et stériliser les matériels.

3. Procédé pour désinfecter et stériliser des matériels infectieux ou contaminés par des microbes, par exemple des instruments médicaux, des déchets de laboratoires médicaux ou d'hôpitaux, les étapes comprenant :
(a) la mise en place du matériel à désinfecter et à stériliser dans une chambre de stérilisation étanche au vide ;
(b) et (c) l'introduction de peroxyde d'hydrogène liquide et d'acide acétique liquide dans un récipient ayant au moins deux compartiments, construits de façon que leur contenu se libère pendant une évacuation ;
(d) l'évacuation du gaz de la chambre de stérilisation, de façon que les deux compartiments distincts des étapes (b) et (c) libèrent leur contenu, de façon à provoquer une évaporation au moins partielle du liquide ;
(e) l'introduction d'ozone gazeux dans la chambre de stérilisation ;
(f) le traitement du matériel dans une chambre de stérilisation pendant un laps de temps suffisant pour désinfecter et stériliser les matériels.

4. Procédé selon la revendication 1, dans lequel les étapes (b) et (c) sont mises en oeuvre dans un ordre quelconque avec production *in situ* d'acide peracétique.

5. Procédé selon la revendication 1, dans lequel les étapes (b) et (c) sont mises en oeuvre simultanément avec production *in situ* d'acide peracétique.

6. Procédé selon l'une des revendications 1 à 5, le procédé étant mis en oeuvre à une température d'environ 15 à 35°C.

7. Procédé selon la revendication 6, qui est mis en oeuvre à la température ambiante.

8. Procédé selon la revendication 3, dans lequel le récipient a un compartiment contenant un catalyseur, pour déplacer vers la droite l'équilibre de l'équation de réaction 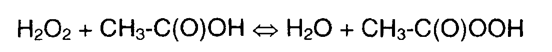 le catalyseur se dégageant en même temps que le peroxyde d'hydrogène et l'acide acétique.

9. Procédé selon la revendication 8, dans lequel le catalyseur est l'acide sulfurique.

10. Procédé selon l'une des revendications 1 à 9, dans lequel, dans l'étape (f), l'ozone et l'acide peracétique formé sont présents dans la phase vapeur.

11. Procédé selon l'une des revendications 1 à 10, dans lequel les étapes (b) à (e) sont effectuées au moins deux fois.

12. Procédé selon l'une des revendications 1 à 11, dans lequel des moyens d'alimentation, pour introduire le peroxyde d'hydrogène, l'acide acétique et l'ozone, sont disposés sur la chambre de stérilisation.

13. Procédé selon l'une des revendications 1 à 12, dans lequel le rapport en moles de l'acide acétique à l'ozone est de 3/1 à 1/3.

14. Procédé selon l'une des revendications 1 à 13, dans lequel l'humidité, dans l'étape (e) ou (f), est d'au moins 10 %.

15. Procédé selon l'une des revendications 1 à 14, dans lequel le matériel est constitué d'un déchet infectieux à l'état solide ou liquide.

16. Procédé selon l'une des revendications 2 à 15 pour traiter des déchets contaminés ou infectieux, dans lequel les déchets sont broyés avant, pendant ou après l'étape (b) ou (c).

17. Procédé selon l'une des revendications 1 à 16, dans lequel l'étape (e) est effectuée au moins deux fois.
